(19) Europäisches Patentamt
European Patent Office
Office européen des brevets

(11) Veröffentlichungsnummer: **0 227 941**
**A1**

(12) **EUROPÄISCHE PATENTANMELDUNG**

(21) Anmeldenummer: 86116192.5

(51) Int. Cl.4: **C07D 237/20** , A61K 31/50

(22) Anmeldetag: 22.11.86

(30) Priorität: 29.11.85 DE 3542197

(43) Veröffentlichungstag der Anmeldung:
08.07.87 Patentblatt 87/28

(84) Benannte Vertragsstaaten:
AT BE CH DE ES FR GB IT LI NL SE

(71) Anmelder: **BASF Aktiengesellschaft**
**Carl-Bosch-Strasse 38**
**D-6700 Ludwigshafen(DE)**

(72) Erfinder: **May, Hans-Joachim, Dr.**
**Birkenweg 40**
**D-6730 Neustadt(DE)**
Erfinder: **Traut, Martin, Dr.**
**Muehltalstrasse 125**
**D-6900 Heidelberg(DE)**
Erfinder: **Weifenbach, Harald, Dr.**
**Londoner Ring 71**
**D-6700 Ludwigshafen(DE)**
Erfinder: **Baldinger, Verena, Dr.**
**Schiffgasse 6**
**D-6900 Heidelberg(DE)**
Erfinder: **Gries, Josef, Dr.**
**Roemerweg 43**
**D-6706 Wachenheim(DE)**
Erfinder: **Teschendorf, Hans-Juergen**
**Georg-Nuss-Strasse 5**
**D-6724 Dudenhofen(DE)**

(54) **Neue 1-Phenyl-4-(1H)-pyridazinimine, ihre Herstellung und Verwendung.**

(57) Es werden 1-Phenyl-4-(1H)-pyridazinimine der Formel

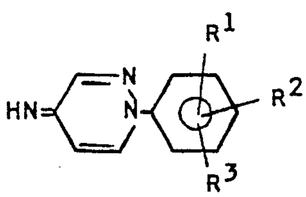
,

worin R', R² und R³ die in der Beschreibung angegebene Bedeutung haben, sowie deren Herstellung beschrieben. Die Verbindungen eignen sich zur Bekämpfung von Krankheiten.

EP 0 227 941 A1

# Neue 1-Phenyl-4-(1H)-pyridazinimine, ihre Herstellung und Verwendung

Die Erfindung betrifft neue, im Phenylring substituierte 1-Phenyl-4-(1H)-pyridazinimine sowie deren Salze mit physiologisch verträglichen Säuren. Weiter betrifft sie Verfahren zur Herstellung dieser Verbindungen sowie deren Verwendung bei der Bekämpfung von Krankheiten.

Es ist bereits eine Reihe von verschiedenartig substituierten 4-(1H)-Pyridazinoniminen beschrieben worden (vgl. BE-PS 645 360, DE-OS 15 42 693 und DE-OS 17 70 772), für die eine Anwendung als Zwischenprodukte für die Herstellung von Farbstoffen, Insektiziden und Pflanzenschutzmitteln oder als Herbizid angegeben wird. Aus den deutschen Offenlegungsschriften 19 12 841, 21 39 687, 22 11 662, 22 45 248, 31 44 138 und 34 36 550 gehen Pyridaziniumsalze mit einem Aminrest in 4-Stellung hervor, die als Zwischenprodukte für die Herstellung von Farbstoffen, Pharmazeutika und Schädlingsbekämpfungsmitteln und teilweise als Arzneimittel mit antidepressiver und blutdrucksteigernder Wirkung beschrieben werden.

Es wurde nun gefunden, daß 1-Phenyl-4-(1H)-pyridazinimine der Formel I

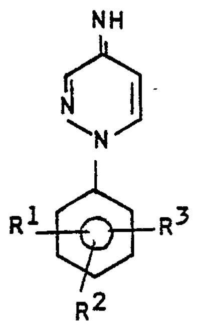

(I),

in der

$R^1$ ein $C_{1-8}$-Alkylrest, ein Cycloalkylrest mit 3 bis 8 C-Atomen im Ring, der gegebenenfalls ein-bis vierfach durch $C_{1-4}$-Alkylreste substituiert ist, ein C $_{1-3}$-Alkoxyrest, ein Trifluormethylrest oder ein Fluor-, o-Chlor-, m-Chlor-oder Bromatom ist, und

$R^2$ und $R^3$, die gleich oder verschieden sein können, Wasserstoff-oder p-Chloratome oder die für $R^1$ genannten Reste bedeuten

sowie deren Salze mit physiologisch verträglichen Säuren, überlegene pharmakologische Eigenschaften aufweisen.

Die Säureadditionssalze der Verbindungen der Formel I können auch als Pyridaziniumsalze gemäß Formel II formuliert

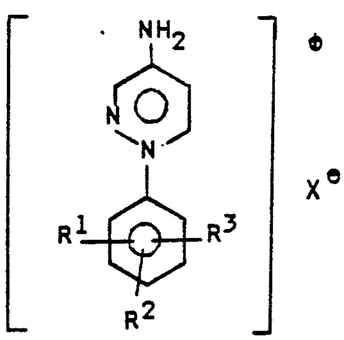

(II)

werden, wobei $X^{\ominus}$ das Säureanion bedeutet.

Als physiologisch verträgliche Säuren kommen die üblichen physiologisch verträglichen anorganischen oder organischen Säuren, wie Chlor-oder Bromwasserstoffsäure, Phosphorsäure, Schwefelsäure, Maleinsäure, Fumarsäure, Milchsäure, Weinsäure, Apfelsäure, Zitronensäure, Salicylsäure, Adipinsäure oder Benzoesäure, in Betracht. Weitere physiologisch verträgliche Säuren können aus Fortschritte der Arzneimittelforschung, Band 10, Seiten 224 -225, Birkhäuser Verlag, Basel und Stuttgart, 1966 oder dem Journal of Pharmaceutical Sciences, Volume 66, Seiten 1 bis 5 (1977) entnommen werden.

Als Verbindungen der Formel I seien im einzelnen solche erwähnt, die folgende Teilstruktur III

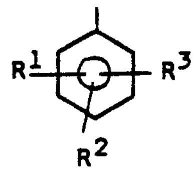

(III)

besitzen:

2-Tolyl, 3-Tolyl, 4-Tolyl, 2-Ethylphenyl, 3-Ethylphenyl, 4-Ethylphenyl, 2-Propylphenyl, 4-Propylphenyl, 4-Isopropylphenyl, 4-Cyclopropylphenyl, 4-Cyclopentylphenyl, 4-Cyclohexylphenyl, 2,3-Dimethylphenyl, 2,4-Dimethylphenyl, 2,5-Dimethylphenyl, 2,6-Dimethylphenyl, 3,4-Dimethylphenyl,3,5-Dimethylphenyl, 2,4,6-Trimethylphenyl, 2,3,5-Trimethylphenyl, 2,3,6-Trimethylphenyl, 2-Methoxyphenyl, 3-Methoxyphenyl, 4-Methoxyphenyl, 2-Ethoxyphenyl, 4-Ethoxyphenyl, 2,3-Dimethoxyphenyl, 2,4-Dimethoxyphenyl, 2,5-Dimethoxyphenyl, 2,6-Dimethoxyphenyl, 3,4-Dimethoxyphenyl, 3,5-Dimethoxyphenyl, 2,3,4-Trimethoxyphenyl, 2,4,5-Trimethoxyphenyl, 2,4,6-Trimethoxyphenyl, 3,4,5-Trimethoxyphenyl, 2-Chlorphenyl, 3-Chlorphenyl, 2-Bromphenyl, 3-Bromphenyl, 4-Bromphenyl, 2-Fluorphenyl, 3-Fluorphenyl, 4-Fluorphenyl, 2,3-Dichlorphenyl, 2,4-Dichlorphenyl, 2,5-Dichlorphenyl, 2,6-Dichlorphenyl, 3,4-Dichlorphenyl, 3,5-Dichlorphenyl, 2,4-Difluorphenyl, 3,4-Di fluorphenyl, 2,6-Difluorphenyl, 2-Chlor-6-fluorphenyl, 3-Chlor-4-fluorphenyl, 4-Chlor-3-fluorphenyl, 3-Brom-4-chlorphenyl, 4-Brom-3-chlorphenyl, 3,4-Dibromphenyl, 2-(Trifluormethyl)phenyl, 3-(Trifluormethyl)-phenyl, 4-(Trifluormethyl)phenyl, 2-Methoxy-5-methylphenyl, 3-Methoxy-4-methylphenyl, 4-Methoxy-2-methylphenyl, 4-Methoxy-3-methylphenyl, 2-Ethoxy-4--ethylphenyl, 2-Chlor-4-methylphenyl, 2-Chlor-5-methylphenyl, 2-Chlor-6--methylphenyl, 3-Chlor-2-methylphenyl, 3-Chlor-4-methylphenyl, 4-Chlor-2--methylphenyl, 4-Chlor-3-methylphenyl, 5-Chlor-2-methylphenyl, 3-Brom-4--methylphenyl, 4-Brom-3-methylphenyl, 2-Chlor-5-methoxyphenyl, 3-Chlor-4-methoxyphenyl, 4-Chlor-3-methoxyphenyl, 5-Chlor-2-methoxyphenyl, 5--Chlor-2-ethoxyphenyl, 3-Fluor-4-methylphenyl, 5-Fluor-2-methylphenyl, 4-Fluor-3-methylphenyl, 3-Chlor-4-(trifluormethyl)-phenyl, 4-Chlor-2-trifluormethyl)-phenyl, 4-Chlor-3-(trifluormethyl)phenyl, 4-Brom-3-(trifluormethyl)phenyl, 4-Fluor-3-(trifluormethyl)phenyl, 2,4,5-Trichlorphenyl, 2--Fluor-4,5-dichlorphenyl, 2-Methyl-4,5-dichlorphenyl, 3-Methyl-4,5-dichlorphenyl und 2-Methyl-3,4-dichlorphenyl.

Bevorzugt sind diejenige Verbindungen der Formel I, in denen $R^3$ Wasserstoff bedeutet. Von diesen sind besonders diejenigen zu nennen, in denen $R^1$ und $R^2$ in der 3-und 4-Stellung des Phenylringes stehen. $R^1$ und $R^2$ sind vorzugsweise Chlor, Brom und/oder Methyl.

Die erfindungsgemäßen 1-Phenyl-4-(1H)-pyridazinimine der Formel I und ihre Salze mit physiologisch verträglichen Säure können hergestellt werden, indem man

a) eine Verbindung der Formel IV

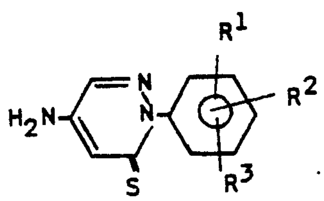

(IV)

mit Wasserstoffperoxid-Lösung in einer organischen Säure umsetzt oder

b) eine Verbindung der Formel IV in Gegenwart von Raney-Nickel hydriert oder

c) eine Verbindung der Formel V

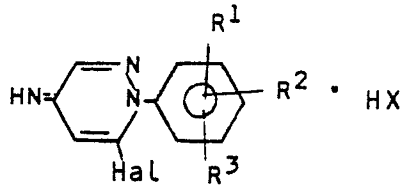

(V)

worin Hal ein Halogenatom und X das Anion einer Säure darstellen, katalytisch hydriert und die so

erhaltenen Verbindungen der Formel I gewünschtenfalls in ihre Salze mit physiologisch verträglichen Säuren überführt.

Die Umsetzung a) erfolgt vorzugsweise in Eisessig als Lösungsmittel bei Temperaturen von 40 bis 150°C, vorzugsweise 70 bis 120°C. Nach Entfernung des Eisessigs kristallisiert dabei das Reaktionsprodukt als Sulfat aus. In der Regel verwendet man 30-gewichtsprozentiges Wasserstoffperoxid; man kann aber auch weniger und höher konzentrierte Wasserstoffperoxid-Lösungen verwenden. Für die Oxidation des Schwefels werden mindestens 3 Mol Wasserstoffperoxid benötigt, ein Überschuß bis zu 500 % schadet nicht. Anstelle von Essigsäure als Lösungsmittel können auch andere Säuren, wie Trifluoressigsäure oder Propionsäure, verwendet werden. Die Reaktion kann unter Normaldruck oder erhöhtem Druck durchgeführt werden.

Die Reaktion b) wird in Gegenwart eines leichten Überschusses einer Mineralsäure, bevorzugt Salzsäure, und in einem inerten Lösungsmittel, bevorzugt Ethanol, bei 20 bis 150°C, bevorzugt bei Rückflußtemperatur des verwendeten Lösungsmittels, durchgeführt. Nach Abfiltrieren des Nickelkatalysators kristallisiert das Reaktionsprodukt, gegebenenfalls nach Entfernen eines Teiles des Lösungsmittels als Hydrochlorid aus, das gegebenenfalls durch Umkristallisation oder durch Freisetzen der Base mit Alkali, vorzugsweise Ammoniak-Lösung, und Überführung in ein anderes Säureadditionssalz einer anorganischen oder organischen Säure gereinigt werden kann.

Die katalytische Dehalogenierung c) gelingt besonders gut mit Verbindungen der Formel V, worin Hal Chlor oder Brom bedeutet. Sie kann mit Wasserstoff als Dehalogenierungsmittel in Gegenwart eines geeigneten Dehalogenierungskatalysators wie Raney-Nickel, Raney-Kobalt, Palladium, Platin und gegebenenfalls in Gegenwart von halogenwasserstoffbindenden Mitteln, wie Natriumacetat, Natriumhydrogencarbonat, Natriumhydroxid, Magnesiumoxid, Calciumhydroxid, Ammoniumhydroxid, Triethylamin, Pyridin vorgenommen werden. Die Dehalogenierung kann unter Normaldruck oder unter Druck bis zu 300 atü und 20°C bis zu 120°C durchgeführt werden. Die Dehalogenierung wird in einem inerten Lösungsmittel oder Suspendiermittel, wie Wasser, einem Ether, wie Diethylether, Tetrahydrofuran oder Dioxan, einem Ester, wie Essigester oder Butylacetat, einem cyclischen aliphatischen oder aromatischen Kohlenwasserstoff, wie Benzol, Toluol oder Cyclohexan, durchgeführt. Die Reaktion kann diskontinuierlich und bei fest angeordnetem Katalysator auch kontinuierlich ausgeführt werden. Die Isolierung der Reaktionsprodukte erfolgt nach der Abtrennung des Katalysators durch Abfiltrieren oder Einengen des Reaktionsgemisches als Hydrohalogenid, bevorzugt als Hydrochlorid oder Hydrobromid. Eine weitere Reinigung kann auf üblichem Wege bevorzugt durch Umkristallisation des Salzes, oder in an sich bekannter Weise durch Freisetzen der freien Verbindungen, z. B. mit Basen oder Ionenaustauschern, vorzugsweise Ammoniumhydroxid-oder Natriumhydroxid-Lösung, und Überführung in ein anderes Säureadditionssalz einer anorganischen oder organischen Säure erfolgen.

Die Ausgangsverbindungen der Formel IV lassen sich beispielsweise nach dem in der japanischen Auslegeschrift 6067/67 beschriebenen Verfahren durch Umsetzung von Pyridazonen-(6) der Formel VI

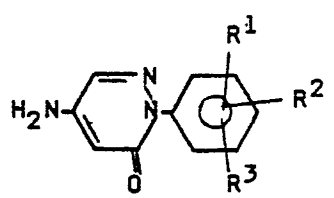

(VI)

mit Phosphorpentasulfid nach M. Yokoyama et. al., Synthesis 1984, 827 bis 829, mit Lawessons-Reagenz - (Bull. Soc. Chim. Belg. 87, 223 (1978)) herstellen. Man kann sie auch nach der DE-OS 16 70 309 aus 6-Halogen-pyridazoniminen-(4) durch Umsetzung mit Schwefelwasserstoff oder in einer Thioharnstoffschmelze als Schwefelwasserstoffquelle gewinnen.

Die Ausgangsverbindungen der Formel V kann man durch Halogenierung von Pyridazonen der Formel VI mit $POCl_3$ oder $POBr_3$ gemäß der in der DE-OS 20 16 691 beschriebenen Weise erhalten. Die Ausgangsverbindungen können auch nach von H. Lundt und P. Lunde in Acta Chemica Scandinavica 21, 1067 bis 1080 (1967) durch Quarternierung geeignet substituierter Pyridazine erhalten werden.

Die Pyridazone der Formel VI sind auf literaturbekannter Weise und nach folgendem Schema zugänglich:

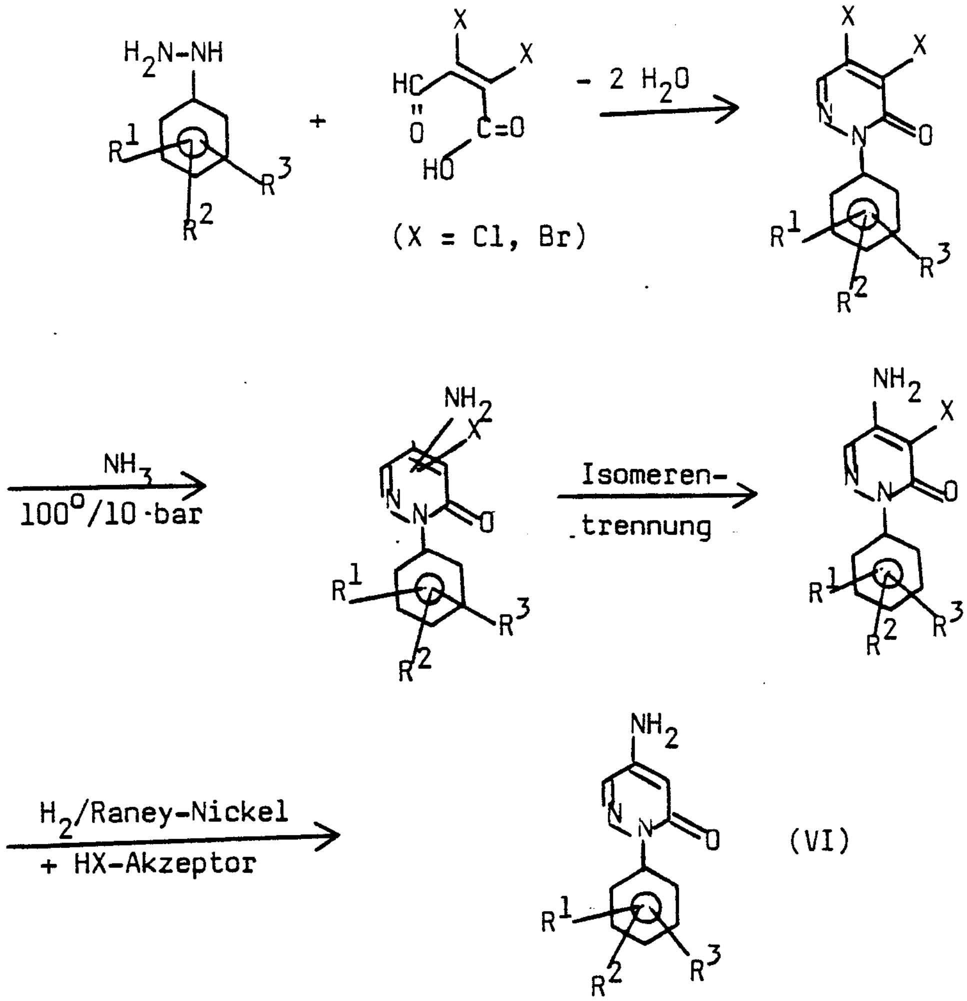


(VI)

Die Verbindungen der Formel I und ihre physiologisch verträglichen Säureadditionssalze weisen wertvolle pharmakologische Eigenschaften auf. Sie wirken vorzugsweise auf zentralnervöse Funktionen und eignen sich als Antidepressiva zur Behandlung von Depressionszuständen sowie als Antiparkinsonmittel zur Behandlung der Parkinson + schen Erkrankung bzw. verwandter Krankheitsbilder.

Die erfindungsgemäßen Substanzen eignen sich zur Behandlung von psychischen Störungen, insbesondere von Depressionen sowie als Mittel zur Behandlung der Parkinson'schen Erkrankung oder vergleichbarer extrapyramidal-motorischer Störungen.

Als Wirkungsmechanismen derzeit therapeutisch genutzter Antidepressiva gelten die Hemmung des neuronalen Re-uptake von Transmittersubstanzen (Noradrenalin und/oder Serotonoin) und die Hemmung der Monoaminoxidase Typ A und/oder Typ B. Eine Hemmung der Monoaminoxidase Typ B gilt auch als Therapieprinzip bei der Parkinson'schen Erkrankung. Es wurden folgende Methoden eingesetzt:

Hemmung der Neurotransmitter-Aufnahme in Rattenhirn-Synaptosomen

Hippocampus und Cortex aus Rattenhirn wurden präpariert und in 0,32 M Sucrose-Lösung homogenisiert. Durch differentielle Zentrifugation wurden Synaptosomen erhalten, die in Pufferlösung suspendiert wurden. Die Synaptosomen sind in der Lage, aus dem umgebenden Medium zugefügte Neurotransmittersubstanzen (z.B. Noradrenalin oder Serotonin) aktiv aufzunehmen. Dieser Vorgang ist durch uptake-Hemmer konzentrationsabhängig antagonisierbar. Die Synaptosomen wurden mit den Testsubstanzen in verschiedenen Konzentrationen versetzt und anschließend mit $^3$H-Noradrenalin (Hippocampus) bzw. $^3$H-Serotonin (Cortex) bei 37°C inkubiert. Die Substratkonzentration betrug ca. 10 nM. Nach Stoppen der Aufnahme durch Verdünnen mit eisgekühlter Pufferlösung wurden die Synaptosomen abzentrifugiert und die $^3$H-Aktivität im Sediment gemessen. Der Blindwert wird durch Inkubation bei 0°C ermittelt.

Aus den verschiedenen Inhibitor-Konzentrationen gegen Kontrolle ermittelten Hemmwerten wurde durch lineare Regression nach logit-log-Transformation die mittlere Hemmkonzentration $IC_{50}$ berechnet.

Hemmung der Monoaminoxidase

Die Bestimmung der Monoaminoxidase erfolgte in verdünntem Rattenhirnhomogenat dem 1. unterschiedliche Konzentrationen der zu prüfenden Testsubstanzen und 2. für die Bestimmung der Monoaminoxidase Typ A $^{14}C$-Tryptamin in einer Konzentration von 0,4 µmol, zur Bestimmung der Monoaminoxidase Typ B $^{14}C$-Phenylethylamin in einer Konzentration von 0,25 µmol zugesetzt wurde. Dieser Ansatz wurde 20 Minuten bei 37°C inkubiert. Die Reaktion wird durch 0,1 n HCl gestoppt und die Reaktionsprodukte nach Extraktion in Toluol-Szintillator (PPO + POPOP in Toluol) bestimmt. Der Blindwert wird in analogen Ansätzen mit einer Inkubationszeit von t = 0 Minuten bestimmt.

Aus den bei den verschiedenen Inhibitorkonzentrationen gegen die Kontrolle ermittelten Hemmwerten wurde durch lineare Regression nach logit-log-Transformation die mittlere Hemmkonzentration ($IC_{50}$) berechnet.

Beeinflussung des Blutdrucks

Da die Vergleichssubstanz (Beisp. 1 der DOS 2245248) eine blutdrucksteigernde Wirkung aufweist, wurden auch die erfindungsgemäßen Substanzen auf diese Wirkungsqualität hin überprüft.

Zur Feststellung der Wirkung auf den arteriellen Blutdruck wurden Sprague-Dawley-Ratten (230 -280 g) mit Urethan (1,78 g/kg i.p.) narkotisiert. Der Blutdruck wurde in der A. carotis gemessen. Die Substanzen wurden i.v. in die V. jugularis appliziert. Als ED20 % wird aus der linearen Regression zwischen log Dosis -(mg/kg) und relativer Blutdruckänderung (Δ %) die Dosis berechnet, die eine 20 %ige Blutdrucksenkung bewirkt.

Die Ergebnisse sind in Tabelle 1 und 2 zusammengestellt. Danach besitzen die Substanzen überraschenderweise gleichzeitig sowohl eine uptake-hemmende als auch eine Monoaminoxidase-hemmende Wirkung, d.h. es werden zwei Wirkprinzipien der Depressionsbehandlung in einer Substanz vereinigt. Die Vergleichssubstanz, für die solche Eigenschaften ebenfalls gefunden wurden, wird in ihrer Wirksamkeit deutlich übertroffen:

-In der Noradrenalin-uptake-Hemmung bis zum 6fachen (Beisp. 17, 18)

-in der Serotonin-uptake-Hemmung bis zu 100 -200fach (Beisp. 1, 16, 17,18)

-in der Hemmung der Monoaminoxidase A bis zum 7fachen (Beisp. 13) und

-in der Hemmung der Monoaminoxidase B bis zum 20 -30fachen (Beisp. 7, 13, 18, 42).

Dagegen tritt die für die Vergleichssubstanz bekannte Blutdrucksteigerung bei den erfindungsgemäßen Verbindungen zurück (Tab. 2). Eine Blutdrucksteigerung ist entweder nicht mehr dosisabhängig vorhanden, so daß eine Berechnung einer ED nicht mehr möglich ist, oder die Substanzen wirken im Gegenteil sogar blutdrucksenkend.

Die erfindungsgemäßen Substanzen sind aufgrund ihrer uptake-und Monoaminoxidase-hemmenden Eigenschaften als Therapeutika für die Depression geeignet, wegen der bei einigen Verbindungen hervortretenden Hemmung der Monoaminoxidase B (Beisp. 7, 42) auch für die Behandlung des Parkinson.

Tab. 1

| Substanz des Beisp. Nr. | Hemmung der Synaptosomen-Aufnahme von | | | | Hemmung der Monoaminoxidase | | | |
|---|---|---|---|---|---|---|---|---|
| | Noradrenalin | | Serotonin | | Typ A | | Typ B | |
| | IC50 (µM) | r.W.* | IC50 (µM) | r.W. | IC50 (µM) | r.W. | IC50 (µM) | r.W. |
| 1 | 0.0058 | 5.3 | 0.0065 | 112 | 1.9 | 2.2 | 2.6 | 18.8 |
| 5 | 0.046 | 0.7 | 0.24 | 1.4 | 1.5 | 2.8 | 10.0 | 4.9 |
| 7 | 0.5 | 0.1 | 0.058 | 12.6 | 0.64 | 6.6 | 1.5 | 32.7 |
| 8 | 0.034 | 0.9 | 0.013 | 56.2 | 2.9 | 1.4 | 10.0 | 4.9 |
| 10 | 0.11 | 0.3 | 0.048 | 15.2 | 1.2 | 3.4 | 10.0 | 4.9 |
| 11 | 0.026 | 1.2 | 0.1 | 7.3 | 2.1 | 2.0 | 5.1 | 9.6 |
| 12 | 0.083 | 0.4 | 0.0087 | 83.9 | 1.8 | 2.3 | 0.7 | 71 |
| 13 | 0.026 | 1.2 | 0.01 | 73.0 | 0.59 | 7.0 | 1.2 | 39.8 |
| 16 | 0.013 | 2.5 | 0.0067 | 109 | 2.14 | 2.0 | 2.7 | 18.1 |
| 17 | 0.0048 | 6.5 | 0.0039 | 184 | 1.53 | 2.7 | 2.6 | 19.0 |
| 18 | 0.0051 | 6.1 | 0.0028 | 261 | 0.87 | 4.8 | 1.9 | 25.8 |
| 32 | 0.7 | 0.1 | 0.044 | 16.6 | 7.0 | 0.6 | 4.0 | 12.2 |
| 42 | 0.015 | 2.1 | 0.02 | 36.5 | 0.75 | 5.6 | 2.0 | 24.5 |
| 45 | 0.017 | 1.8 | 0.018 | 40.6 | 1.0 | 4.2 | 5.0 | 9.8 |
| 46 | 0.01 | 3.1 | 0.0071 | 103 | 1.0 | 4.2 | 4.0 | 12.2 |
| Beisp. Nr. 1 d. DE-OS 2245248 | 0.031 | 1 | 0.73 | 1 | 4.2 | 1 | 49 | 1 |

* r.W. = relative Wirksamkeit (= Quotient $\frac{\text{IC50 Referenzverbindung}}{\text{IC50 Beispielverbindung}}$ )

Tab. 2

| Substanz des Beispiels Nr. | Beeinflussung des Blutdrucks bei narkotisierten Ratten: Erhöhung Dosis (mg/kg i.v.) → Effekt prozentuale Änderung (Δ %) | Erniedrigung Dosis (mg/kg i.v.) → Effekt prozentuale Änderung (Δ %) |
|---|---|---|
| 5 | | 0.1 → 10 |
| 7 | | 2.15 → 12 |
| 10 | | 1 → 13 |
| 11 | | 1 → 11 |
| 12 | | 1 → 5 |
| 16 | 0.1 → 20 | |
| 17 | 0.1 → 10 | |
| 18 | | 0.1 → 7 |
| 32 | | 1 → 7 |
| 42 | 0.1 → 20 | |
| 45 | 0.1 → 20 | |
| 46 | 0.1 → 20 | |
| Beisp. Nr. 1 der DE-OS 2245248 | 0.22* | |

* ED 20 %

Gegenstand der vorliegenden Erfindung sind demnach auch therapeutische Mittel oder Zubereitungen, die neben pharmazeutisch üblichen Träger-und Verdünnungsmitteln eine Verbindung der Formel I oder eines ihrer physiologisch verträglichen Säureadditionssalze als Wirkstoff enthalten, sowie die Verwendung der neuen Verbindungen zu therapeutischen Zwecken.

Die erfindungsgemäßen Verbindungen können in üblicher Weise oral oder parenteral (subkutan, intravenös, intramuskular, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 0,03 und 15 mg/kg Körpergewicht bei oraler Gabe und zwischen etwa 0,003 und 1,5 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gewichtsprozent.

Beispiel 1

1-(3,4-Dichlorphenyl)-4-(1H)-pyridazinimin-hydrochlorid

a) 1-(3,4-Dichlorphenyl)-4,5-dibrom-6-pyridazon:

213,5 g (1 Mol) 3,4-Dichlorphenylhydrazinhydrochlorid, 83 g (1 Mol) Natriumacetat und 257,9 g (1 Mol) Mucobromsäure wurden in 3 l Eisessig 3 h unter Rückfluß gekocht, anschließend bei 100°C tropfenweise mit 350 ml Wasser versetzt und auf Raumtemperatur abgekühlt. Nach Absaugen des Kristallisates, Waschen mit Wasser und Trocknen erhielt man 321,5 g (80,6 %) 1-(3,4-Dichlorphenyl)-4,5-dibrom-6-pyridazon, Fp. 208 -209°C.

b) 1-(3,4-Dichlorphenyl)-4-amino-5-brom-6-pyridazon:

69 g (173 mmol) 1-(3,4-Dichlorphenyl)-4,5-dibrom-6-pyridazon, 300 ml Methanol und 500 ml Wasser wurden in einen HC-Autoklaven gegeben, 250 g Ammoniak aufgepreßt und 20 h bei Eigendruck (ca. 10 bar) auf 100°C erhitzt. Nach dem Abkühlen und Entspannen des Autoklaven wurde das Reaktionsgemisch mit 1 l Wasser versetzt, das ausgefallene Produkt abgesaugt, mit Wasser gewaschen und getrocknet. Man erhielt 45,8 g (79,0 %) Rohprodukt, Fp. 278 -280°C. Dieses wurde zur Entfernung von geringen Anteilen des falschen Isomeren 1-(3,4-Dichlorphenyl)-4-brom-5-amino-6-pyridazon zweimal mit siedendem Chloroform digeriert. Als Rückstand verblieben 40,5 g (69,9 %) 1-(3,4-Dichlorphenyl)-4-amino-5-brom-6-pyridazon, Fp. 286 -288°C.

c) 1-(3,4-Dichlorphenyl)-4-amino-6-pyridazon:

33,5 g (100 mmol) 1-(3,4-Dichlorphenyl)-4-amino-5-brom-6-pyridazon und 8,6 g (105 mmol) wasserfreies Natriumacetat wurden in 800 ml Ethanol suspendiert, 9,3 g Raney-Nickel unter Stickstoff zugegeben und anschließend unter Rühren bei Normaldruck und Raumtemperatur mit Wasserstoff begast, bis die theoretische Menge von 2,24 l Wasserstoff aufgenommen war. Nach dem Abfiltrieren des Katalysators wurde das Filtrat zur Trockne eingeengt, in Wasser suspendiert, abgenutscht, der Filterrückstand mit Wasser gewaschen und anschließend getrocknet. Man erhielt 24,7 g (96,5 %) 1-(3,4-Dichlorphenyl)-4-amino-6-pyridazon, Fp. 252 -253,5°C.

d) 1-(3,4-Dichlorphenyl)-4-amino-6-pyridazinthion:

25,6 g (100 mmol) 1-(3,4-Dichlorphenyl)-4-amino-6-pyridazon wurden in 500 ml wasserfreiem Toluol suspendiert, 30,3 g (75 mmol) Lawesson-Reagens [2,4-Bis-(4-methoxyphenyl)-2,4-dithioxo-1,3,2,4-dithiadiphosphetan] zugegeben und 12 h unter Rühren gekocht. Nach dem Abkühlen wurde abgesaugt, mit Toluol gewaschen und getrocknet. Man erhielt 25,2 g (96,3 %) rohes 1-(3,4-Dichlorphenyl)-4-amino-6-pyridazinthion, Fp. 252 -255°C, das für die weiteren Umsetzungen genügend rein war. Eine aus Ethanol umkristallisierte Probe schmolz bei Fp. 260,5 -262°C.

e) 1-(3,4-Dichlorphenyl)-4-(1H)-pyridazinimin-hydrochlorid:

27,2 g (100 mmol) 1-(3,4-Dichlorphenyl)-4-amino-6-pyridazinthion wurden in 725 ml Ethanol suspendiert, 54,2 g wasserfeuchtes Raney-Nickel zugegeben, zum Sieden erhitzt, unter Rühren innerhalb von 2 h 64,6 g konzentrierte Salzsäure zugetropft und weitere 4 h unter Rückfluß gekocht. Der Ansatz wurde heiß filtriert, der Filterrückstand mit Ethanol ausgekocht und die Filtrate vereinigt. Beim Abkühlen erhielt man 21,3 g rohes 1-(3,4-Dichlorphenyl)-4-(1H)-pyridazinimin-hydrochlorid, Fp. 306,5 -308°C. Aus der Mutterlauge können nach Einengen weitere 2,7 g Rohprodukt erhalten werden (Gesamtrohausbeute 86,8 %). Nach Umkristallisation aus Methanol unter Aktivkohlezusatz erhielt man 20,8 g (75,2 %) reines 1-(3,4-Dichlorphenyl)-4-(1H)-pyridzinimin-hydrochlorid vom Fp. 316,5 -318°C.

Beispiel 2

1-(3,4-Dichlorphenyl)-4-(1H)-pyridazinimin-fumarat

a) 1-(3,4-Dichlorphenyl)-4-(1H)-pyridazinimin-sulfat:

27,2 g (100 mmol) 1-(3,4-Dichlorphenyl)-4-amino-6-pyridazinthion (vgl. Beispiel 1 d) wurden in 200 ml Eisessig auf 40°C erwärmt, unter Rühren tropfenweise mit 22,6 g (200 mmol) 30-prozentiger Wasserstoffperoxidlösung versetzt und 4 h bei 110 bis 120°C gehalten. Nach dem Abkühlen wurde am Rotationsverdampfer zur Trockene eingeengt, der Rückstand mit Essigester digeriert, das Lösungsmittel abdekantiert und der halbkristalline Rückstand in wenig heißem Ethanol gelöst. Beim Abkühlen schieden sich 14,1 g - (41,7 %) 1-(3,4-Dichlorphenyl)-4-(1H)-pyridazinimin-sulfat, Fp. 199 -202°C ab.

b) 1-(3,4-Dichlorphenyl)-4-(1H)-pyridazinimin-fumarat:

8,5 g (25 mmol) 1-(3,4-Dichlorphenyl)-4-(1H)-pyridazinimin-sulfat wurden in Wasser gelöst, mit überschüssiger Ammoniumhydroxid-Lösung versetzt und mit Dichlormethan extrahiert. Nach dem Trocknen über wasserfreiem Natriumsulfat und Abziehen des Lösungsmittels erhielt man 1-(3,4-Dichlorphenyl)-4-(1H)-pyridazinimin als freie Base, Fp. 128 -129°C. Diese wurde in wenig Ethanol gelöst und eine Lösung von 2,9 g (25 mmol) Fumarsäure in Ethanol zugegeben. Die ausgefallenen Kristalle wurden abgesaugt. Man erhielt 7,9 g (88,7 %) 1-(3,4-Dichlorphenyl)-4-(1H)-pyridazinimin-fumarat, Fp. 219 -220°C.

Beispiel 3

1-(3,4-Dichlorphenyl)-4-(1H)-pyridazinimin-hydrobromid:

a) 1-(3,4-Dichlorphenyl)-6-brom-4-(1H)-pyridazinimin-hydrobromid:

25,6 g (100 mmol) 1-(3,4-Dichlorphenyl)-4-amino-6-pyridazon (vgl. Beispiel 1 c) wurden mit 198 ml geschmolzenem Phosphorylbromid versetzt und 12 h bei 110 -120°C gerührt. Der erkaltete Rückstand wurde dreimal mit absolutem Ether digeriert, das Kristallisat anschließend unter Feuchtigkeitsausschluß mit absolutem Ether gewaschen und im Vakuum bei 40°C über $P_2O_5$-Trockenmittel getrocknet. Man erhielt in quantitativer Ausbeute 1-(3,4-Dichlorphenyl)-6-brom-4-(1H)-pyridazinimin-hydrobromid als stark hygroskopische Kristalle.

b) 1-(3,4-Dichlorphenyl)-4-(1H)-pyridazinimin-hydrobromid:

40,0 g (100 mmol) rohes 1-(3,4-Dichlorphenyl)-6-brom-4-(1H)-pyridazinimin-hydrobromid wurden in 1 l absolutem Tetrahydrofuran gelöst, mit 8,6 g (105 mmol) wasserfreiem Natriumacetat und 6 g 10 % Pd-Aktivkohle-Kontakt versetzt und bei Normaldruck und Raumtemperatur unter Rühren mit Wasserstoff begast, bis die theoretische Menge (2,24 l) aufgenommen war. Die Reaktionslösung wurde filtriert, das Filtrat zur Trockene eingeengt und der Rückstand aus Ethanol unter Aktivkohle-Zusatz umkristallisiert. Man erhielt 16,3 g (50,8 %) 1-(3,4--Dichlorphenyl)-4-(1H)-pyridazinimin-hydrobromid, Fp. 310 -312°C.

Beispiel 4

1-(2-Methylphenyl)-4-(1H)-pyridazinimin-hydrochlorid:

a) 1-(2-Methylphenyl)-4,5-dichlor-6-pyridazon:

Diese Substanz wurde analog Beispiel 1 a) aus o-Tolylhydrazin-hydrochlorid und Mucochlorsäure mit 85,9 % Ausbeute hergestellt, Fp. 119 -121°C.

b) 1-(2-Methylphenyl)-4-amino-5-chlor-6-pyridazon:

Diese Substanz wurde analog Beispiel 1 b) aus 1-(2-Methylphenyl)-4,5-dichlor-6-pyridazon mit 77,6 % Rohausbeute erhalten, Fp. 203 -211°C. Nach Isomerentrennung wurden 49,4 % reine Substanz erhalten, Fp. 226 -228°C.

c) 1-(2-Methylphenyl)-4-amino-6-pyridazon:

Diese Substanz wurde analog Beispiel 1 c) aus 1-(2-Methylphenyl)-4-amino-5-chlor-6-pyridazon mit 1,0 g (5 Mol-proz.) Palladium(II)chlorid als Dehalogenierungskatalysator (anstelle von Raney-Nickel) mit 88,5 % Ausbeute erhalten, Fp. 173,5 -175°C.

d) 1-(2-Methylphenyl)-6-chlor-4-(1H)-pyridazinimin-hydrochlorid:

Diese Substanz wurde analog Beispiel 2 a) aus 1-(2-Methylphenyl)-4-amino-6-pyridazon mit Phosphorylchlorid (anstelle von Phosphorylbromid) mit 51,6 % Ausbeute erhalten, Fp. 237 -238,5°C.

e) 1-(2-Methylphenyl)-4-(1H)-pyridazinimin-hydrochlorid:

Diese Substanz wurde analog Beispiel 2 b) aus 1-(2-Methylphenyl)-6-chlor-4-(1H)-pyridazinimin-hydrochlorid mit 49,7 % Ausbeute erhalten, Fp. 242 -243°C.

Beispiel 5

1-(4-Methylphenyl)-4-(1H)-pyridazinimin-hydrobromid

a) 1-(4-Methylphenyl)-4-amino-6-pyridazon:

Diese Substanz wurde analog Beispiel 1 c) aus 1-(4-Methylphenyl)-4-amino-5-brom-6-pyridazon erhalten, Ausbeute 94,2 %, Fp. 232,5 -235°C.

b) 1-(4-Methylphenyl)-6-brom-4-(1H)-pyridaziniminhydrobromid:

Diese Substanz wurde analog Beispiel 2 a) in quantitativer Ausbeute erhalten. Sie ist stark hygroskopisch.

c) 1-(4-Methylphenyl)-4-(1H)-pyridazinimin-hydrobromid:
Diese Substanz wurde analog Beispiel 2 b) erhalten, Ausbeute 40,3 %, Fp. 232 -233°C.

Nach dem in Beispiel 1 beschriebenen Verfahren wurden die folgenden erfindungsgemäßen Substanzen hergestellt:

Beispiel 6:

Aus 1-(3-Methylphenyl)-4-amino-6-pyridazinthion (Fp. 205 -206°C): 1-(3-Methylphenyl)-4-(1H)-pyridazinimin-hydrochlorid, Fp. 260 -261,5°C.

Beispiel 7:

Aus 1-(4-Ethylphenyl)-4-amino-6-pyridazinthion (Fp. 212 -214°C): 1-(4-Ethylphenyl)-4-(1H)-pyridazinimin-hydrochlorid, Fp. 236 - 237°C.

Beispiel 8:

Aus 1-(4-Bromphenyl)-4-amino-6-pyridazinthion (Fp. 292 -295°C): 1-(4-Bromphenyl)-4-(1H)-pyridazinimin-hydrochlorid, Fp. 245 -247°C.

Beispiel 9:

Aus 1-(2,4-Dimethylphenyl)-4-amino-6-pyridazinthion (Fp. 222 -224°C): 1-(2,4-Dimethylphenyl)-4-(1H)-pyridazinimin-hydrochlorid, Fp. 268,5 -270°C.

Beispiel 10:

Aus 1-(3-Methoxy-4-methylphenyl)-4-amino-6-pyridazinthion (Fp. 210 -212,5°C): 1-(3-Methoxy-4-methylphenyl)-4-(1H)-pyridazinimin-hydrochlorid, Fp. 268 -269°C.

Beispiel 11:

Aus 1-(3-Fluor-4-methylphenyl)-4-amino-6-pyridazinthion (Fp. 240 -242,5°C): 1-(3-Fluor-4-methylphenyl)-4-(1H)-pyridazinimin-hydrochlorid, Fp. 252 -253°C.

Beispiel 12:

Aus 1-(3-Chlor-4-trifluormethylphenyl)-4-amino-6-pyridazinthion (Fp. 228 -231°C): 1-(3-Chlor-4-trifluormethylphenyl)-4-(1H)-pyridazinimin-hydrochlorid, Fp. 285,5 - 287°C (kristallisiert mit 0,25 Mol Kristall-Ethanol).

Beispiel 13:

Aus 1-(3-Brom-4-methylphenyl)-4-amino-6-pyridazinthion (Fp. 258 -260°C): 1-(3-Brom-4-methylphenyl)-4-(1H)-pyridazinimin-hydrochlorid, Fp. 280,5 -281,5°C (kristallisiert mit 0,25 Mol Kristall-Methanol).

Beispiel 14:

Aus 1-(3-Trifluormethyl-4-fluorphenyl)-4-amino-6-pyridazinthion (Fp. 237,5 -241 °C): 1-(3-Trifluormethyl-4-fluorphenyl)-4-(1H)-pyridazinimin-hydrochlorid, Fp. 260 -261 °C.

Beispiel 15:

Aus 1-(3-Trifluormethyl-4-chlorphenyl)-4-amino-6-pyridazinthion (Fp. 186 -188°C): 1-(3-Trifluormethyl-4-chlorphenyl)-4-(1H)-pyridazinimin-hydrochlorid, Fp. 283 -284°C.

Beispiel 16:

Aus 1-(3-Brom-4-chlorphenyl)-4-amino-6-pyridazinthion (Fp. 256 -258°C): 1-(3-Brom-4-chlorphenyl)-4-(1H)-pyridazinimin-hydrochlorid, Fp. 298 -299,5°C.

Beispiel 17:

Aus 1-(3-Chlor-4-bromphenyl)-4-amino-6-pyridazinthion (Fp. 260 -262°C): 1-(3-Chlor-4-bromphenyl)-4-(1H)-pyridazinimin-hydrochlorid, Fp. 316,5 -318°C.

Beispiel 18:

Aus 1-(3,4-Dibromphenyl)-4-amino-6-pyridazinthion (Fp. 251 -253°C): 1-(3,4-Dibromphenyl)-4-(1H)-pyridazinimin-hydrochlorid, Fp. 310 -311°C.

Beispiel 19:

Aus 1-(2-Methyl-4,5-dichlorphenyl)-4-amino-6-pyridazinthion (Fp. 217 -219°C): 1-(2-Methyl-4,5-dichlorphenyl)-4-(1H)-pyridazinimin-hydrochlorid, Fp. 330°C.

Beispiel 20:

Aus 1-(2,4-Dichlorphenyl)-4-amino-6-pyridazinthion (Fp. 172 -176°C): 1-(2,4-Dichlorphenyl)-4-(1H)-pyridazinimin-hydrochlorid, Fp. 276,5 -278°C.

Beispiel 21:

Aus 1-(3-Chlor-4-methoxyphenyl)-4-amino-6-pyridazinthion (Fp. 242,5 -245°C): 1-(3-Chlor-4-methoxyphenyl)-4-(1H)-pyridazinimin-hydrochlorid, Fp. 256 -257°C.

Nach dem in Beispiel 1 beschriebenen Verfahren können zum Beispiel hergestellt werden: 1-(2-Methoxyphenyl)-4-(1H)-pyridazinimin-hydrochlorid, 1-(3-Methoxyphenyl)-4-(1H)-pyridazinimin-hydrochlorid, 1-(4-Methoxyphenyl)-4-(1H)-pyridazinimin-hydrochlorid, 1-(3,4-Dimethoxyphenyl)-4-(1H)-pyridzainimin-hydrochlorid, 1-(3,4,5-Trimethoxyphenyl)-4-(1H)-pyridazinimin-hydrochlorid, 1-(4-Isopropylphenyl)-4-(1H)-pyridazinimin-hydrochlorid, 1-(4-Cyclohexylphenyl)-4-(1H)-pyridazinimin-hydrochlorid, 1-(2,3-Dichlorphenyl)-4-(1H)-pyridazinimin-hydrochlorid, 1-(3,5-Dichlorphenyl)-4-(1H)-pyridazinimin-hydrochlorid, 1-(2,4,5-Trichlorphenyl)-4-(1H)-pyridazinimin-hydrochlorid, 1-(2-Methyl-3,4-dichlorphenyl)-4-(1H)-pyridazinimin-hydröchlorid, 1-(3-Methyl-4,5-dichlorphenyl)-4-(1H)-pyridazinimin-hydrochlorid, 1-(2-Chlor-6-methylphenyl)-4-(1H)-pyridaziniminhydrochlorid, 1-(3-Fluor-4-chlorphenyl)-4-(1H)-pyridazinimin-hydrochlorid.

Nach dem in Beispiel 2 b) beschriebenen Verfahren wurden die folgenden Säureadditionssalze des 1-(3,4-Dichlorphenyl)-4-(1H)-pyridazinimin herhergestellt:

Beispiel 22:

1-(3,4-Dichlorphenyl)-4-(1H)-pyridazinimin-succinat, Fp. 180 -181°C.

Beispiel 23:

1-(3,4-Dichlorphenyl)-4-(1H)-pyridazinimin-malat, Fp. 118 -120°C.

Beispiel 24:

1-(3,4-Dichlorphenyl)-4-(1H)-pyridazinimin-tartrat, Fp. 168 -170°C.

Beispiel 25:

1-(3,4-Dichlorphenyl)-4-(1H)-pyridazinimin-maleat, Fp. 183 -184°C.

Nach dem in Beispiel 3 beschriebenen Verfahren wurden die folgenden erfindungsgemäßen Substanzen synthetisiert:

Beispiel 26:

1-(2-Fluorphenyl)-4-(1H)-pyridazinimin-hydrobromid, Fp. 211 -213°C (kristallisiert mit 0,5 Mol Kristallwasser).

Beispiel 27:

1-(4-Fluorphenyl)-4-(1H)-pyridazinimin-hydrobromid, Fp. 240 -241,5°C.

Beispiel 28:

1-(2-Chlorphenyl)-4-(1H)-pyridazinimin-hydrobromid, Fp. 234 -236°C.

Beispiel 29:

1-(3-Chlorphenyl)-4-(1H)-pyridazinimin-hydrobromid, Fp. 222,5 -224°C.

Beispiel 30:

1-(2-Trifluormethylphenyl)-4-(1H)-pyridazinimin-hydrobromid, Fp. 235 -237°C.

Beispiel 31:

1-(3-Trifluormethylphenyl)-4-(1H)-pyridazinimin-hydrobromid, Fp. 238 -239,5°C.

Beispiel 32:

1-(4-Trifluormethylphenyl)-4-(1H)-pyridazinimin-hydrobromid, Fp. 234 -235,5°C.

Beispiel 33:

1-(2,3-Dimethylphenyl)-4-(1H)-pyridazinimin-hydrobromid, Fp. 271,5 -273°C.

Beispiel 34:

1-(2,6-Dimethylphenyl)-4-(1H)-pyridazinimin-hydrobromid, Fp. 289 -291,5°C (kristallisiert mit 0,5 mol Kristallwasser).

Beispiel 35:

1-(2-Methyl-4-chlorphenyl)-4-(1H)-pyridazinimin-hydrobromid, Fp. 305 -306°C.

Beispiel 36:

1-(2-Ethoxy-5-chlorphenyl)-4-(1H)-pyridazinimin-hydrobromid, Fp. 199 -200°C.

Beispiel 37:

1-(2,5-Dichlorphenyl)-4-(1H)-pyridazinimin-hydrobromid, Fp. 284,5 -286°C.

Beispiel 38:

1-(2,6-Dichlorphenyl)-4-(1H)-pyridazinimin-hydrobromid, Fp. 248 -250°C (kristallisiert mit 0,25 Mol Kristallwasser).

Beispiel 39:

1-(2,4,6-Trimethylphenyl)-4-(1H)-pyridazinimin-hydrobromid, Fp. 292,5 -294°C.

Nach dem in Beispiel 4 d) und e) beschriebenen Verfahren wurden die folgenden erfindungsgemäßen Substanzen hergestellt:

Beispiel 40:

Aus 1-(3,4-Dimethylphenyl)-6-chlor-4-(1H)-pyridazinimin-hydrochlorid (Fp. 249 -251°C): 1-(3,4-Dimethylphenyl)-4-(1H)-pyridazinimin-hydrochlorid, Fp. 300°C.

Beispiel 41:

Aus 1-(3-Methyl-4-fluorphenyl)-6-chlor-4-(1H)-pyridazinimin-hydrochlorid (Fp. 264 -266°C): 1-(3-Methyl-4-fluorphenyl)-4-(1H)-pyridazinimin-hydrochlorid, Fp. 300 -301°C.

Beispiel 42:

Aus 1-(3-Chlor-4-methylphenyl)-6-chlor-4-(1H)-pyridazinimin-hydrochlorid (Fp. 205 -209°C): 1-(3-Chlor-4-methylphenyl)-4-(1H)-pyridazinimin-hydrochlorid, Fp. 273,5 - 275°C.

Beispiel 43:

Aus 1-(3,4-Difluorphenyl)-6-chlor-4-(1H)-pyridazinimin-hydrochlorid (Fp. 262,5 -264°C): 1-(3,4-Difluorphenyl)-4-(1H)-pyridazinimin-hydrochlorid, Fp. 251 -252,5°C.

Beispiel 44:

Aus 1-(3-Chlor-4-fluorphenyl)-6-chlor-4-(1H)-pyridazinimin-hydrochlorid (Fp. 273,5 - 275°C): 1-(3-Chlor-4-fluorphenyl)-4-(1H)-pyridazinimin-hydrochlorid, Fp. 307 - 309°C.

Nach der in Beispiel 2 a) beschriebenen Arbeitsweise wurde weiterhin hergestellt:

Beispiel 45:

Aus 1-(3-Methyl-4-chlorphenyl)-4-amino-6-pyridazinthion (Fp. 282 -285°C): 1-(3-Methyl-4-chlorphenyl)-4-(1H)-pyridazinimin-sulfat, Fp. 206 -208°C.

Beispiel 46:

Aus 1-(3-Methyl-4-bromphenyl)-4-amino-6-pyridazinthion (Fp. 278 -280,5°C): 1-(3-Methyl-4-bromphenyl)-4-(1H)-pyridazinimin-sulfat, Fp. 182,5 -184°C.

Formulierungsbeispiele:

Beispiel 47:

Tabletten:

| | |
|---|---|
| Wirkstoff, gemäß Formel I | 10 mg |
| Polyvinylpyrrolidon (mittl. M.G. 25.000) | 170 mg |
| Polyethylenglykol (mittl. M.G. 4.000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10-prozentiger wäßriger Lösung befeuchtet, durch ein Sieb mit der lichten Maschenweite 1,0 mm getrieben und bei 50°C getrocknet. Dieses Granulat wird mit Polyethylenglykol (mittl. M.G. 4 000), Hydroxypropylmethylcellulose, Talkum und Magnesiumstearat vermischt und zu Tabletten a 240 mg verpreßt.

Beispiel 48

| Dragees: | |
|---|---|
| Wirkstoff, gemäß Formel I | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8-prozentigen wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

## Ansprüche

1. 1-Phenyl-4-(1H)-pyridazinimine der Formel I

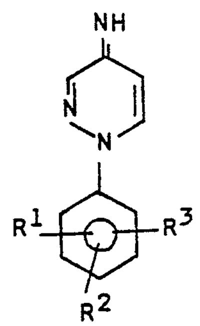

(I),

in der
$R^1$ ein $C_{1-8}$-Alkylrest, ein Cycloalkylrest mit 3 bis 8 C-Atomen im Ring, der gegebenenfalls ein-bis vierfach durch $C_{1-4}$-Alkylreste substituiert ist, ein C $_{1-3}$-Alkoxyrest, ein Trifluormethylrest oder ein Fluor-, o-Chlor-, m-Chlor-oder Bromatom ist, und
$R^2$ und $R^3$, die gleich oder verschieden sein können, Wasserstoff-oder p-Chloratome oder die für $R^1$ genannten Reste bedeuten
sowie deren Salze mit physiologisch verträglichen Säuren.

2. 1-Phenyl-4-(1H)-pyridazinimine der in Anspruch 1 angegebenen Formel, in der $R^3$ Wasserstoff bedeutet.

3. 1-Phenyl-4-(1H)-pyridazinimine der in Anspruch 1 angegebenen Formel, in der $R^1$ die 3-und $R^2$ die 4-Position des Phenylringes besetzen.

4. 1-Phenyl-4-(1H)-pyridazinimine nach Anspruch 3, in denen $R^1$ und $R^2$, gleich oder verschieden sind und Chlor, Brom oder Methyl bedeuten.

5. 1-(3,4-Dichlorphenyl)-4-(1H)-pyridazinimin und seine physiologisch verträglichen Säureadditionssalze.

6. 1-(4-Brom-3-chlorphenyl)-4-(1H)-pyridazinimin und seine physiologisch verträglichen Säureadditionssalze.

7. 1-(3-Brom-4-chlorphenyl)-4-(1H)-pyridazinimin und seine physiologisch verträglichen Säureadditionssalze.

8. 1-(3-Methyl-4-bromphenyl)-4-(1H)-pyridazinimin und seine physiologisch verträglichen Säureadditionssalze.

9. Verbindung der Formel I gemäß Anspruch 1 zur Verwendung bei der Bekämpfung von Krankheiten.

10. Verwendung der Verbindungen der Formel I bei der Bekämpfung von Störungen des ZNS.

11. Verfahren zur Herstellung der 1-Phenyl-4-(1H)-pyridazinimine der Formel I

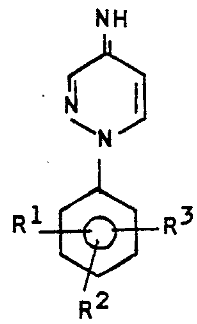

(I),

in der

$R^1$ ein $C_{1-8}$-Alkylrest, ein Cycloalkylrest mit 3 bis 8 C-Atomen im Ring, der gegebenenfalls ein-bis vierfach durch $C_{1-4}$-Alkylreste substituiert ist, ein $C_{1-3}$-Alkoxyrest, ein Trifluormethylrest oder ein Fluor-, o-Chlor-, m-Chlor-oder Bromatom ist, und

$R^2$ und $R^3$, die gleich oder verschieden sein können, Wasserstoff-oder p-Chloratome oder die für $R^1$ genannten Reste bedeuten

sowie deren Salze mit physiologisch verträglichen Säuren, dadurch gekennzeichnet, daß man

a) eine Verbindung der Formel IV

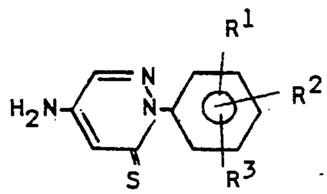

(IV)

mit Wasserstoffperoxid-Lösung in einer organischen Säure umsetzt oder

b) eine Verbindung der Formel IV in Gegenwart von Raney-Nickel hydriert oder

c) eine Verbindung der Formel V

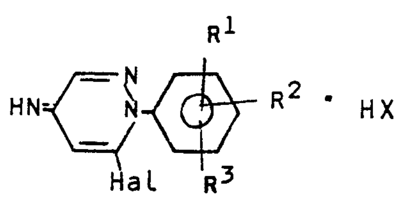

(V)

worin Hal ein Halogenatom und X das Anion einer Säure darstellen, katalytisch hydriert

und die so erhaltenen Verbindungen der Formel I gewünschtenfalls in ihre Salze mit physiologisch verträglichen Säuren überführt.

Patentansprüche für folgenden Vertragsstaaten : AT;ES

1. Verfahren zur Herstellung der 1-Phenyl-4-(1H)-pyridazinimine der Formel I

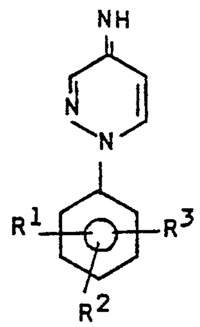

(I),

in der

$R^1$ eine $C_{1-8}$ -Alkylrest, ein Cycloalkylrest mit 3 bis 8 C-Atomen im Ring, der gegebenfalls ein-bis vierfach durch $C_{1-4}$-Alkylreste substituiert ist, eine $C_{1-3}$-Alkoxyrest, ein Trifluormethylrest oder ein Fluor-, o-Chlor-, m-Chlor-oder Bromatom ist, und

$R^2$ und $R^3$, die gleich oder verschieden sein können, Wasserstoff-oder p-Chloratome oder die für $R^1$ genannten Reste bedeuten

sowie deren Salze mit physiologisch verträglichen Säuren, dadurch gekennzeichnet, daß man

a) eine Verbindung der Formel IV

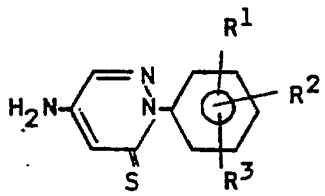

(IV)

mit Wasserstoffperoxid-Lösung in einer organischen Säure umsetzt oder

b) eine Verbindung der Formel IV in Gegenwart von Raney-Nickel hydriert oder

c) eine Verbindung der Formel V

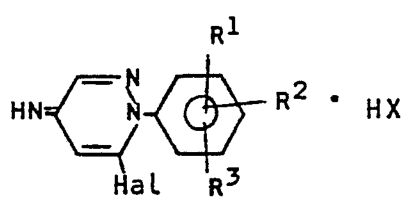

(V)

worin Hal ein Halogenatom und X das Anion einer Säure darstellen, katalytisch hydriert

und die so erhaltenen Verbindungen der Formel I gewünschtenfalls in ihre Salze mit physiologisch verträglichen Säuren überführt.

2. Verfahren zur Herstellung von 1-Phenyl-4-(1H)-pyridazinimine der in Anspruch 1 angegebenen Formel, in der $R^3$ Wasserstoff bedeutet.

3. Verfahren zur Herstellung von 1-Phenyl-4-(1H)-pyridazinimine der in Anspruch 1 angegebenen Formel, in der $R^1$ die 3-und $R^2$ die 4-Position des Phenylringes besetzen.

4. Verfahren zur Herstellung von 1-Phenyl-4-(1H)-pyridazinimine nach Anspruch 3, in denen $R^1$ und $R^2$, gleich oder verschieden sind und Chlor, Brom oder Methyl bedeuten.

5. Verfahren zur Herstellung von 1-(3,4-Dichlorphenyl)-4-(1H)-pyridazinimin und seinen physiologisch verträglichen Säureadditionssalzen.

6. Verfahren zur Herstellung von 1-(4-Brom-3-chlorphenyl)-4-(1H)-pyridazinimin und seinen physiologisch verträglichen Säureadditionssalzen.

7. Verfahren zur Herstellung von 1-(3-Brom-4-chlorphenyl)-4-(1H)-pyridazinimin und seinen physiologisch verträglichen Säureadditionssalzen.

8. Verfahren zur Herstellung von 1-(3-Methyl-4-bromphenyl)-4-(1H)-pyridazinimin und seinen physiologisch verträglichen Säureadditionssalzen.

9. Verbindung der Formel I gemäß Anspruch I zur Verwendung bei der Bekämpfung von Krankheiten.

10. Verwendung der Verbindungen der Formel I bei der Bekämpfung von Störungen des ZNS.

Europäisches Patentamt

# EUROPÄISCHER RECHERCHENBERICHT

Nummer der Anmeldung

EP 86 11 6192

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 078 989 (BASF)<br>* Seiten 1,2,84,90 * | 1,9,10 | C 07 D 237/20<br>A 61 K 31/50 |
| D,A | DE-A-1 542 693 (BASF)<br>* Seiten 1-3,8,9 * | 1 | |
| | | | RECHERCHIERTE SACHGEBIETE (Int. Cl.4) |
| | | | C 07 D 237/00 |

Der vorliegende Recherchenbericht wurde für alle Patentansprüche erstellt.

| Recherchenort | Abschlußdatum der Recherche | Prüfer |
|---|---|---|
| DEN HAAG | 14-04-1987 | FRANCOIS J.C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82